# EUROPÄISCHE PATENTSCHRIFT

(11) **EP 2 807 450 B1**
(45) Veröffentlichungstag und Bekanntmachung des Hinweises auf die Patenterteilung: **26.02.2020**
(21) Anmeldenummer: 13706441.6
(22) Anmeldetag: 28.01.2013
(51) Int. Cl.: A61B 5/107, A61C 9/00, A61C 19/04, A61C 13/34, G01B 21/04, G06T 7/80, G09B 23/28

(54) **VERFAHREN UND REFERENZMODELL ZUR ÜBERPRÜFUNG EINES VERMESSUNGSSYSTEMS**
METHOD AND REFERENCE MODEL FOR CHECKING A MEASURING SYSTEM
PROCÉDÉ ET MODÈLE DE RÉFÉRENCE PERMETTANT DE VÉRIFIER UN SYSTÈME DE MESURE

(30) Priorität: 27.01.2012 DE 102012201193
(43) Veröffentlichungstag der Anmeldung: 03.12.2014
(73) Patentinhaber: Sirona Dental Systems GmbH, 64625 Bensheim (DE)
(72) Erfinder: POPILKA, Björn, 69502 Hemsbach (DE); WEDLER, Volker, 69493 Hirschberg (DE); ADAMSON, Anders, 64297 Darmstadt (DE); THIEL, Frank, 64372 Ober-Ramstadt (DE)
(74) Vertreter: Özer, Alpdeniz
(86) Internationale Anmeldenummer: PCT/EP2013/051547
(87) Internationale Veröffentlichungsnummer: WO 2013/110806

(56) Entgegenhaltungen:
- EP-A2- 0 895 192
- WO-A1-2011/106472
- WO-A2-2010/077380
- US-A- 4 073 071

## Beschreibung

### Technisches Gebiet

Die Erfindung betrifft ein Verfahren zur Überprüfung eines Vermessungssystems, wobei mehrere dreidimensionale Aufnahmen mittels des Vermessungssystems von einem Referenzobjekt aus unterschiedlichen Aufnahmerichtungen aufgenommen werden.

### Stand der Technik

Aus dem Stand der Technik sind mehrere Verfahren zur Überprüfung von Vermessungssystemen bekannt.

US 4,073,071 offenbart ein Zahnmodell bestehend aus einem Oberkiefer-Modell und einem Unterkiefer-Modell, die durch Scharniere verbunden sind.

WO 2010/077380 A2 offenbart ein Verfahren zur Registrierung von optischen Aufnahmen einer Zahnsituation, wobei bei einer globalen Registrierung zusätzlich zu den Aufnahmen aus okklusaler Richtung zusätzliche Aufnahmen aus bukkaler Richtung bzw. lingualer Richtung gemacht werden, um die Registrierung zu verbessern und Registrierungsfehler zu minimieren. Zur Korrektur eines akkumulierten Registrierungsfehlers wird also beispielsweise eine zusätzliche bukkale Aufnahme mittels des optischen Scanners durchgeführt.

Bei einem weiteren Verfahren wird im ersten Schritt ein Referenzmodell mittels eines präzisen Laborsystems vermessen und dabei ein 3D-Modell des Referenzmodells erzeugt. Anschließend wird im zweiten Schritt das gleiche Referenzmodell mittels des zu prüfenden Vermessungssystems vermessen und ein zweites 3D-Modell erzeugt. Daraufhin wird das erste mit dem Laborsystem vermessene 3D-Modell mit dem zweiten 3D-Modell verglichen. Die Differenz zwischen den beiden 3D-Modellen zeigt dann eine fehlerhafte Kalibrierung bzw. Registrierung auf. Die Differenz zwischen den 3D-Modellen kann beispielsweise durch Falschfarbenmarkierungen angezeigt werden. Es kann auch ein Mittelwert der Abweichung gebildet werden, indem die Differenz für mehrere Messpunkte entlang eines normalen Vektors für die gesamte Oberfläche des 3D-Modells gemittelt wird.

Bei einem weiteren Verfahren kann ein Referenzmodell mit bekannten Abmessungen mittels des zu überprüfenden Vermessungssystems vermessen werden, wobei ein 3D-Modell erzeugt wird. Dieses Differenzmodell kann beispielsweise ein Modell eines Oberkiefers oder eines Unterkiefers sein. Nach der Vermessung wird beispielsweise der Abstand zwischen Kauflächen der letzten Zähne des Oberkiefers bzw. des Unterkiefers im 3D-Modell vermessen und mit dem tatsächlichen Abstand zwischen den beiden letzten Zähnen des Referenzmodells verglichen. Eine Abweichung der beiden Abstände weist auf einen Registrierungsfehler und/oder einen Kalibrierungsfehler hin.

Ein Nachteil dieses Verfahrens ist, dass die Abmessungen des Referenzmodells, wie der Abstand zwischen den letzten Zähnen des Oberkiefers bzw. des Unterkiefers, entweder genau vermessen werden oder bereits bekannt sein müssen, um einen Vergleich zu ermöglichen. Die Markierung mit Falschfarben und die Bestimmung eines Mittelwerts der Abweichung ist mit einem hohen technischen Aufwand verbunden und erschwert es dem Benutzer festzustellen, ob die Genauigkeit der Kalibrierung und/oder der Registrierung innerhalb erlaubten Toleranzgrenzen liegt oder nicht.

Die Aufgabe der vorliegenden Erfindung besteht daher darin, ein Verfahren und ein Referenzmodell zur Überprüfung eines Vermessungssystems bereitzustellen, die eine einfache Überprüfung eines Vermessungssystems ohne größeren technischen Aufwand ermöglichen.

### Darstellung der Erfindung

Die Erfindung betrifft ein Verfahren zur Überprüfung eines Vermessungssystems, wobei mehrere dreidimensionale Aufnahmen mittels des Vermessungssystems von einem Referenzobjekt aus unterschiedlichen Aufnahmerichtungen aufgenommen werden. Das Referenzobjekt weist dabei eine geschlossene Form auf. Jede der dreidimensionalen Aufnahmen wird mindestens mit der vorangehenden Aufnahme registriert, wobei bei einer fehlerhaften Kalibrierung und/oder bei einer fehlerhaften Registrierung die einzelnen Aufnahmen im Vergleich zur tatsächlichen Form des Referenzobjekts verformt sind, sodass sich beim Zusammensetzen der einzelnen dreidimensionalen Aufnahmen zu einer Gesamtaufnahme die Verformung fortsetzt und dadurch die erzeugte Gesamtaufnahme in ihren Abmessungen von den Abmessungen des Referenzobjekts abweicht. Dabei wird mindestens ein Objektbereich des Referenzobjekts am Anfang eines Umlaufs und am Ende des Umlaufs doppelt vermessen. Zwischen einer ersten Position des Objektbereichs in einer ersten Aufnahme am Anfang des Umlaufs und einer zweiten Position des Objektbereichs in einer zweiten Aufnahme am Ende des Umlaufs in der Gesamtaufnahme wird dann ein Abstand bestimmt.

Erfindungsgemäß wird anschließend unter Verwendung dieses Abstandes ein Messfehler des Vermessungssystems bestimmt, der einen Kalibrierungsfehler und/oder einen Registrierungsfehler umfasst.

Das Vermessungssystem zur dreidimensionalen optischen Messung kann beispielsweise auf einem Streifenprojektionsverfahren, auf einem konfokalen optischen Verfahren oder auf einem Farbstreifenprojektionsverfahren beruhen. Das zu prüfende Vermessungssystem kann auch auf einem Röntgen-Computertomografieverfahren (CT) beruhen. Bei einem optischen dreidimensionalen Vermessungssystem werden mehrere dreidimensionale optische Aufnahmen vom Referenzobjekt aus unterschiedlichen Aufnahmerichtungen erzeugt und anschließend werden die einzelnen Aufnahmen zu einer Gesamtaufnahme zusammengesetzt. Bei einem CT-Vermessungssystem werden mehrere Röntgen-Projektionsaufnahmen aus mehreren Aufnahmevorrichtungen vom Referenzobjekt durchgeführt. Anschließend werden die einzelnen Röntgenprojektionsaufnahmen unter Verwendung einer computergestützten Bildrekonstruktion zu einer dreidimensionalen Röntgenaufnahme des Referenzobjekts zusammengesetzt.

Eine Aufnahme wird durch den Benutzer ausgelöst, im zweiten Schritt wird die Dentalkamera relativ zum Referenzobjekt weiterbewegt und anschließend eine zweite Aufnahme ausgelöst. Auf diese Weise werden mehrere Aufnahmen aus unterschiedlichen Aufnahmerichtungen durchgeführt, bis das Referenzobjekt vollständig erfasst ist. Dadurch weisen die Aufnahmebereiche der Aufnahmen unterschiedliche Abstände zueinander auf, die variieren können.

Die Vermessung kann auch unter Verwendung eines Drehtellers erfolgen, wobei das Referenzobjekt für jede Aufnahme um einen bestimmten Winkelabschnitt gedreht wird. Auf diese Weise kann die Vermessung unter gleichen Bedingungen wiederholt werden. Denn die Aufnahmebereiche der einzelnen Aufnahmen weisen durch die festgelegten Aufnahmerichtungen definierte Überlappungsbereiche auf, sodass das Ergebnis der Registrierung wiederholbar ist. Der Drehteller kann entsprechend mittels einer Steuerungsvorrichtung angesteuert werden und mit dem Vermessungssystem synchronisiert werden. Dadurch wird eine automatisierte Vermessung unter festgelegten Bedingungen ermöglicht.

Die geschlossene Form des zur Durchführung des Verfahrens verwendeten Referenzobjekts kann beispielsweise kreisförmig, ovalförmig oder die Form einer beliebigen dreidimensionalen Schleife aufweisen. Beispielsweise kann die geschlossene Form dem Verlauf eines herkömmlichen Artikulators mit einem Oberkiefer, einem Unterkiefer und den Verbindungsstellen zwischen dem Oberkiefer und dem Unterkiefer entsprechen. Erfindungsgemäß ist das Referenzmodell zur Überprüfung eines dentalen Systems aus mehreren Zähnen aufgebaut und ist in seiner Form kreisförmig. Ein herkömmlicher Artikulator ist nicht beansprucht. Bei der Registrierung wird jede der Aufnahmen mit der vorangehenden Aufnahme registriert. Zusätzlich kann jede der Aufnahmen mit der vorvorangehenden Aufnahme registriert werden, die vor der vorangehenden Aufnahme angeordnet ist. Dadurch wird das Ergebnis der Registrierung zusätzlich verbessert. Voraussetzung für diese zusätzliche Registrierung ist jedoch, dass die drei Aufnahmen gemeinsame Überlappungsbereiche aufweisen.

Die Registrierung erfolgt in herkömmlicher Weise durch Verwendung bekannter Registrierungsalgorithmen, wobei übereinstimmende Strukturen in den Aufnahmen erkannt und zusammengeführt werden. Bei fehlerhaften Algorithmen kann die Registrierung fehlerhaft sein und den Registrierungsfehler verursachen. Das Vorliegen eines solchen Registrierungsfehlers kann mittels des vorliegenden Verfahrens erkannt werden. Ein Registrierungsfehler kann auch durch verrauschte Aufnahmen oder durch Aufnahme mit einer sehr geringen Auflösung verursacht werden.

Die Registrierung erfolgt Aufnahme um Aufnahme entlang der gesamten geschlossenen Form des Referenzobjekts bis zum Ausgangspunkt der Registrierung. Falls die Registrierung fehlerhaft ist, weicht eine erste Position des ersten Aufnahmebereichs von der Position desselben Aufnahmebereichs im Verlauf der Registrierung des gesamten Referenzobjekts ab. Für den Benutzer ist dadurch sofort klar erkennbar, dass das Vermessungssystem ein Messfehler aufweist.

Die Vermessung des Referenzobjekts und die anschließende Registrierung kann auch in mehr als einem Umlauf entlang der geschlossenen Form erfolgen. Der zu vergleichende Aufnahmebereich am tatsächlichen Referenzobjekt kann beliebig ausgewählt werden. Es kann auch ein bestimmter charakteristischer Punkt am Referenzobjekt zum Vergleich verwendet werden. Dieser ausgewählte Aufnahmebereich oder der charakteristische Punkt wird dann unter Verwendung bekannter Registrierungsalgorithmen am Anfang eines Umlaufs und am Ende eines Umlaufs entlang der geschlossenen Form ermittelt, sodass ein Abstand zwischen dem zu vergleichenden Aufnahmebereich bzw. dem charakteristischen Punkt bestimmt werden kann. Dieser Abstand gibt das Ausmaß des Messfehlers des Vermessungssystems an.

Der Messfehler kann zusätzlich zum Registrierungsfehler auch durch den Kalibrierungsfehler verursacht werden. Der Kalibrierungsfehler kann beispielsweise durch fehlerhafte Einstellungen der Kameraparameter des Vermessungssystems verursacht werden. Bei einem Vermessungssystem auf der Grundlage des Streifenprojektionsverfahrens sind die maßgeblichen Kameraparameter der Abstand zwischen der Kamera und dem Referenzobjekt, der Einfallswinkel sowie eine Gitterperiode eines Gitters zur Erzeugung eines Streifenmusters.

Die Kameraparameter können auch auf einem Lochkameramodell beruhen, wobei zwischen den intrinsischen und extrinsischen unterschieden wird. Mögliche intrinsische Parameter sind beispielsweise die Brennweite der Kamera, die Pixelkoordinate der Bildmitte und Verzeichnisparameter. Die extrinsischen Parameter können die Rotation und die Translation zwischen Kamera und Projektor umfassen.

Die einzelnen Aufnahmen können auch in kurzen Abständen hintereinander beispielsweise mit mehr als 10 Hz erfolgen. Dadurch ist eine sogenannte Überflugmessung möglich, bei der die handgehaltene Dentalkamera gleichmäßig über das vermessende Referenzobjekt bewegt wird und währenddessen ihre Aufnahmen aus unterschiedlichen Aufnahmerichtungen mit Aufnahmebereichen durchgeführt werden, die für die Registrierung ausreichend große Überlappungsbereiche aufweisen. Das Referenzobjekt, das zur Durchführung des Verfahrens verwendet wird, ist mit dreidimensionalen Objekten versehen, die zur eindeutigen Registrierung geeignet sind. Diese Objekte können beispielsweise beliebige geometrische Formen, wie Würfel, Tetraeder oder Halbkreise sein, die ungeordnet verteilt sind. Erfindungsgemäß sind die Objekte Modelle von Zähnen, die aneinandergereiht sind, wobei das Referenzmodell zur Überprüfung eines dentalen Systems in seiner Form ringförmig ist.

Ein Vorteil dieses Verfahrens liegt darin, dass zur Überprüfung des Vermessungssystems die Abmessungen des Referenzobjekts nicht bekannt sein müssen. Der Messfehler wird lediglich durch den Vergleich eines Aufnahmebereichs am Anfang eines Umlaufs und am Ende eines Umlaufs in der erzeugten Gesamtaufnahme ermittelt. Im Vergleich zu Verfahren, bei denen die Aufnahmen des Vermessungssystems mit Musteraufnahmen eines kalibrierten Laborsystems verglichen werden, besteht der Unterschied darin, dass beim vorliegenden Verfahren ein möglicher Messfehler eines solchen Laborsystems ausgeschlossen wird.

Vorteilhafterweise kann das Referenzobjekt ringförmig sein und Modelle von mehreren aneinandergereihten Zähnen aufweisen.

Damit werden realistische Messbedingungen des Vermessungssystems, nämlich die Vermessung von Zähnen eines Oberkiefers oder eines Unterkiefers, nachgeahmt. Die ringförmige Anordnung der Zähne kann beispielsweise alle Zähne eines Oberkiefers oder eines Unterkiefers in der richtigen Reihenfolge umfassen. Das heißt, dass die beiden letzten Backenzähne zueinander benachbart sind.

Vorteilhafterweise kann eine Trajektorie des Vermessungssystems beim Aufnehmen der Aufnahmen aus unterschiedlichen Aufnahmerichtungen kreisförmig um das ringförmige Referenzobjekt verlaufen.

Dadurch werden mehrere Aufnahmen aus Aufnahmerichtungen mit bestimmten Winkelabständen zueinander durchgeführt, so dass die Überlappungsbereiche der Aufnahmebereiche für die Registrierung ausreichend sind.

Vorteilhafterweise kann das Referenzobjekt einem herkömmlichen Artikulator mit einem Oberkiefer und einem Unterkiefer entsprechen, der eine geschlossene Form aufweist.

Dadurch kann als Referenzobjekt ein herkömmlicher Artikulator verwendet werden, der einen Oberkiefer, einen Unterkiefer und Verbindungsstege zwischen dem Oberkiefer und dem Unterkiefer aufweist. Das Vermessungssystem wird dann entlang des Verlaufes dieses Artikulators geführt, wobei mehrere Aufnahmen erzeugt werden, solange bis der gesamte Artikulator erfasst wird.

Vorteilhafterweise kann eine Trajektorie des Vermessungssystems beim Aufnehmen der Aufnahmen aus unterschiedlichen Aufnahmerichtungen entlang der Form des Artikulators verlaufen.

Dadurch wird der gesamte Artikulator vollständig erfasst, sodass eine Registrierung durchgeführt werden kann.

Vorteilhafterweise kann die Aufnahmerichtung einer letzten Aufnahme in der Reihenfolge der Aufnahmen mit der Aufnahmerichtung einer ersten Aufnahme übereinstimmen.

Dadurch wird zur Durchführung des Verfahrens lediglich ein Umlauf benötigt. Für den Vergleich werden dann die erste Aufnahme und die letzte Aufnahme herangezogen, wobei der zu vergleichende Aufnahmebereich unter Verwendung der Mustererkennungsverfahren in der ersten Aufnahme und in der letzten Aufnahme erkannt wird und ein Abstand zwischen der ersten Position des Aufnahmebereichs oder des charakteristischen Punkts in der ersten Aufnahme und des Aufnahmebereichs oder des charakteristischen Punkts in der letzten Aufnahme bestimmt wird.

Vorteilhafterweise kann ein erster Aufnahmebereich des Referenzobjekts einer ersten Aufnahme in der Reihenfolge der Aufnahmen zumindest teilweise mit einem Aufnahmebereich einer der nachfolgenden Aufnahmen übereinstimmen.

Dadurch erfolgt die Vermessung in mehr als einem Umlauf, sodass manche Aufnahmebereiche doppelt vermessen werden. Diese am Anfang eines Umlaufs und am Ende eines Umlaufs vermessenen Aufnahmebereiche können für den Vergleich herangezogen werden.

Vorteilhafterweise kann vor der Durchführung der dreidimensionalen Aufnahmen der Registrierungsfehler bestimmt werden, indem aus einem ersten ursprünglichen virtuellen 3D-Modell des Referenzobjekts virtuelle einzelne Teilbereiche nacheinander ausgeschnitten werden, wobei jeder Teilbereich mit einem vorangehenden Teilbereich und mit einem nachfolgenden Teilbereich teilweise überlappend ist. Anschließend wird erfindungsgemäß jeder Teilbereich mit mindestens einem vorangehenden Teilbereich registriert, sodass ein zweites virtuelles 3D-Modell des Referenzobjekts nach der Registrierung erzeugt wird. Aus einer Abweichung zwischen dem ersten ursprünglichen 3D-Modell und dem zweiten 3D-Modell des Referenzobjekts wird dann der Registrierungsfehler bestimmt.

Dadurch wird der Registrierungsfehler bereits vor der Vermessung bestimmt, indem Teilbereiche aus dem idealen ersten 3D-Modell virtuell ausgeschnitten und registriert werden. Anschließend kann auch der Kalibrierungsfehler bestimmt werden, indem der Registrierungsfehler vom Messfehler abgezogen wird.

Vorteilhafterweise können die Teilbereiche, die aus dem ersten ursprünglichen 3D-Modell ausgeschnitten werden, in ihren Abmessungen den Aufnahmebereichen der einzelnen durchzuführenden Aufnahmen entsprechen.

Dadurch wird die Registrierung bei der Vermessung möglichst genau simuliert. Denn bei abweichenden Überlappungsbereichen kann die Registrierung zu einem anderen Ergebnis führen.

Erfindungsgemäß wird der Kalibrierungsfehler bestimmt, indem der Registrierungsfehler vom gesamten Messfehler abgezogen wird.

Dadurch wird ersichtlich, ob der Messfehler eher durch die fehlerhafte Registrierung oder durch die fehlerhafte Kalibrierung verursacht wird.

Vorteilhafterweise kann jede Aufnahme mit der vorangehenden Aufnahme und mit der vorvorangehenden Aufnahme teilweise überlappend sein, wobei jede Aufnahme mit der vorangehenden Aufnahme und mit der vorvorangehenden Aufnahme registriert wird.

Dadurch wird die Registrierung zusätzlich verbessert. Voraussetzung dafür ist, dass die Aufnahmebereiche einander so nahe sind, dass jede Aufnahme mit der vorangehenden und mit der vorvorangehenden Aufnahme Überlappungsbereiche aufweist. Die Registrierung kann auch mit der vorvorvorangehenden Aufnahme erfolgen, falls diese übereinstimmende Übergangsbereiche aufweist.

Die Erfindung betrifft weiterhin ein Referenzmodell zur Überprüfung eines dentalen Vermessungssystems zur Vermessung dreidimensionaler Aufnahmen. Das Referenzmodell ist aus mehreren aneinander gereihten Modellen von Zähnen entlang des gesamten Umfangs des Referenzmodells aufgebaut und weist eine geschlossene Form auf. Das Referenzmodell ist in seiner Form ringförmig.

Dieses Referenzmodell ist zur Durchführung des oben genannten Verfahrens geeignet. Im Unterschied zu herkömmlichen Referenzmodellen, die den Abmessungen eines Oberkiefers oder eines Unterkiefers entsprechen, weist das vorliegende Referenzmodell eine geschlossene ringförmige Form auf. Die geschlossene Form hat den Vorteil, dass bei der Vermessung des Referenzmodells das Vermessungssystem nach einem Umlauf wieder zum Ausgangspunkt gelangt, sodass eine erste Aufnahme am Anfang eines Umlaufs mit einer zweiten Aufnahme am Ende eines Umlaufs auf einfache Art und Weise verglichen werden kann, um einen Messfehler festzustellen.

Durch das ringförmige Referenzmodell verläuft die Vermessung entlang einer kreisförmigen Trajektorie. Eine solche Vermessung kann beispielsweise mittels eines automatisierten Drehtellers, auf dem das Referenzobjekt angebracht ist, realisiert werden.

Vorteilhafterweise können die Modelle mehreren Zähnen eines Oberkiefers und/oder eines Unterkiefers entsprechen. Dadurch kann das dentale Vermessungssystem unter realistischen Vermessungsbedingungen überprüft werden.

Das vorliegende Verfahren zur Überprüfung von Vermessungssystemen ist insbesondere zur Qualitätskontrolle bei der Herstellung solcher Vermessungssysteme beim Hersteller geeignet. Das vorliegende Verfahren kann jedoch auch für eine Nachprüfung solcher Vermessungssysteme beim Konsumenten, beispielsweise in einer Zahnarztpraxis, verwendet werden. Dazu benötigt der Benutzer lediglich das oben beschriebene Referenzobjekt, das mittels des Vermessungssystems vermessen wird. Der Benutzer kann zur Durchführung des Verfahrens auch eine zusätzliche Software benötigen, die die Abweichung und den Messfehler bestimmt. Dadurch kann jedes Vermessungssystem auf einfache Art und Weise nachgerüstet werden, um das vorliegende Verfahren durchzuführen.

### Kurzbeschreibung der Zeichnungen

Die Erfindung wird anhand der Zeichnungen erläutert. Es zeigt, die
- Fig. 1: eine Skizze zur Verdeutlichung des vorliegenden Verfahrens, die
- Fig. 2: zeigt eine Seitenansicht des Referenzobjekts umfassend einen Schneidezahn, die
- Fig. 3: zeigt ein alternatives als solches nicht beanspruchtes Referenzobjekt, die
- Fig. 4: zeigt eine Skizze zur Verdeutlichung einer Alternative des vorliegenden Verfahrens mit einem herkömmlichen Artikulator, die
- Fig. 5: zeigt eine Skizze des gesamte Vermessungssystems.

### Ausführungsbeispiel

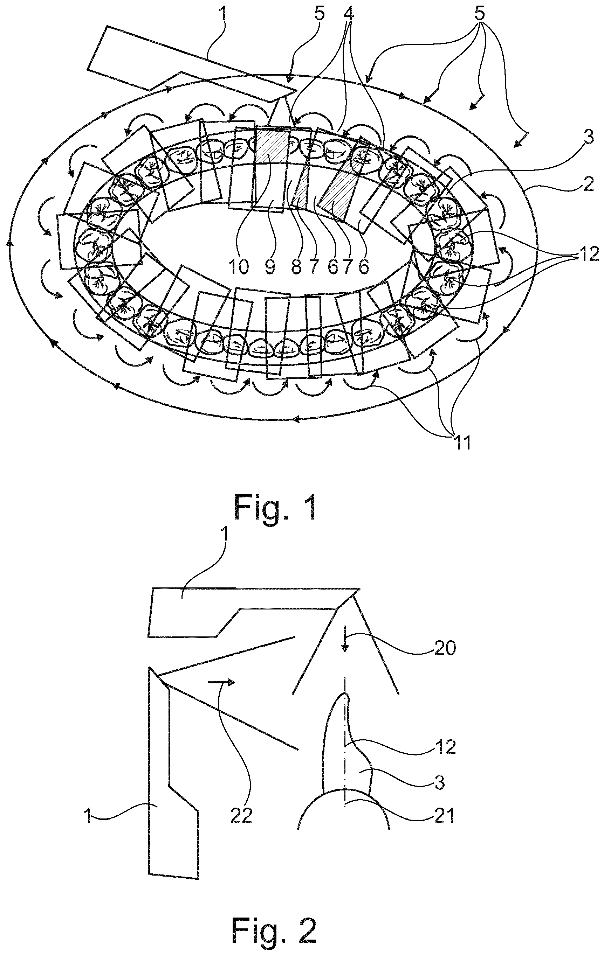

Die Fig. 1 zeigt eine Skizze zur Verdeutlichung des vorliegenden Verfahrens zur Überprüfung eines Vermessungssystems 1, wie einer Dentalkamera. Die Dentalkamera 1 kann nach einem beliebigen dreidimensionalen Vermessungssystem, beispielsweise nach einem Streifenprojektionsverfahren oder nach einem konfokalen Vermessungssystem funktionieren. Zur Durchführung des Verfahrens wird das Vermessungssystem 1 entlang einer Trajektorie 2 um ein Referenzobjekt 3 bewegt, wobei mehrere dreidimensionale Aufnahmen 4 aus unterschiedlichen Aufnahmerichtungen 5, die durch Pfeile anzeigt sind, aufgenommen werden. Das Vermessungssystem 1 umläuft dabei entlang der Trajektorie 2 mindestens einmal das Referenzobjekt 3. Die Aufnahmebereiche 6 der Aufnahmen 4 sind im vorliegenden Fall rechteckig und weisen Überlappungsbereiche 7 zum jeweils vorangehenden Aufnahmebereich auf, die schraffiert dargestellt sind. Es wird also zunächst eine erste Aufnahme mit einem ersten Aufnahmebereich 8 aufgenommen und nach einem Umlauf eine letzte Aufnahme dieses Umlaufs mit einem zweiten Aufnahmebereich 9 aufgenommen, wobei ein zu vergleichender Objektbereich 10 des Referenzobjekts 3, der schraffiert dargestellt ist, sowohl in der ersten Aufnahme 8 des Umlaufs als auch in der letzten Aufnahme 9 des Umlaufs enthalten ist. Jede der Aufnahmen 4 wird mindestens mit der vorangehenden Aufnahme registriert, wie durch die Pfeile 11 angedeutet ist. Falls die Aufnahmebereiche 6 nahe genug beieinander sind, kann jede Aufnahme zusätzlich zur Registrierung mit der vorangehenden Aufnahme mit der vorvorangehenden Aufnahme registriert werden. Die Registrierung verläuft also Aufnahme für Aufnahme bis alle einzelnen Aufnahmen 4 zu einer Gesamtaufnahme des gesamten Referenzobjekts zusammengesetzt sind. Falls die Registrierung und/oder die Kalibrierung des Vermessungssystems fehlerhaft sind, ist jede der dreidimensionalen Aufnahmen 4 im Vergleich zu den tatsächlichen Abmessungen des Referenzobjekts 3 leicht verformt, sodass sich diese Verformung beim Zusammensetzen der einzelnen Aufnahmen 4 fortsetzt. Unter Verwendung dieser Abweichung zwischen der Gesamtaufnahme und den tatsächlichen Abmessungen des Referenzobjekts 3 wird anschließend der Messfehler bestimmt, der einen Kalibrierungsfehler und/oder einen Registrierungsfehler umfasst. Im vorliegenden Fall ist das Referenzobjekt ringförmig und ist aus mehreren aneinander gereihten Modellen von Zähnen 12 gebildet, wobei die obere Hälfte des Referenzmodells Zähne eines Oberkiefers und die untere Hälfte des Referenzmodells Zähne eines Unterkiefers umfasst. Das Referenzmodell kann auch andere dreidimensionale Modelle umfassen, die zur Registrierung geeignet sind. Wesentlich ist dabei, dass die dreidimensionalen Modelle kein widerkehrendes Muster bilden und eindeutig identifizierbar sind. Die Vermessung kann auch über einen Umlauf hinaus erfolgen, sodass mehrere Objektbereiche des Referenzobjekts 3 doppelt vermessen werden. In solch einem Fall kann einer dieser Objektbereiche für den Vergleich und die Bestimmung des Messfehlers herangezogen werden. Der Vorteil dieses Verfahrens liegt darin, dass für die Bestimmung des Messfehlers die tatsächlichen Abmessungen des Referenzobjekts nicht bekannt sein müssen.

Die Fig. 2 zeigt eine Seitenansicht des Referenzobjekts 3 umfassend einen Schneidezahn 12. Die Vermessung mittels der Dentalkamera 1 erfolgt in einem ersten Schritt aus einer ersten Aufnahmerichtung 20, die in etwa parallel zu einer Zahnachse 21 des Zahns 12 verläuft und anschließend in einem zweiten Schritt das Referenzobjekt 3 aus einer zweiten Aufnahmerichtung 22 vermessen wird. Dadurch wird die dreidimensionale Form des Referenzobjekts 3 sowohl von oben aus der ersten Aufnahmerichtung 20 als auch von der Seite aus der zweiten Aufnahmerichtung 22 erfasst. Auf diese Weise wird das gesamte Referenzobjekt 3 Zahn für Zahn entlang eines Umlaufs vermessen. Der Vorteil der zusätzlichen seitlichen Vermessung liegt darin, dass die Registrierung durch eine größere zu registrierende Oberfläche des Referenzobjekts 3 verbessert wird.

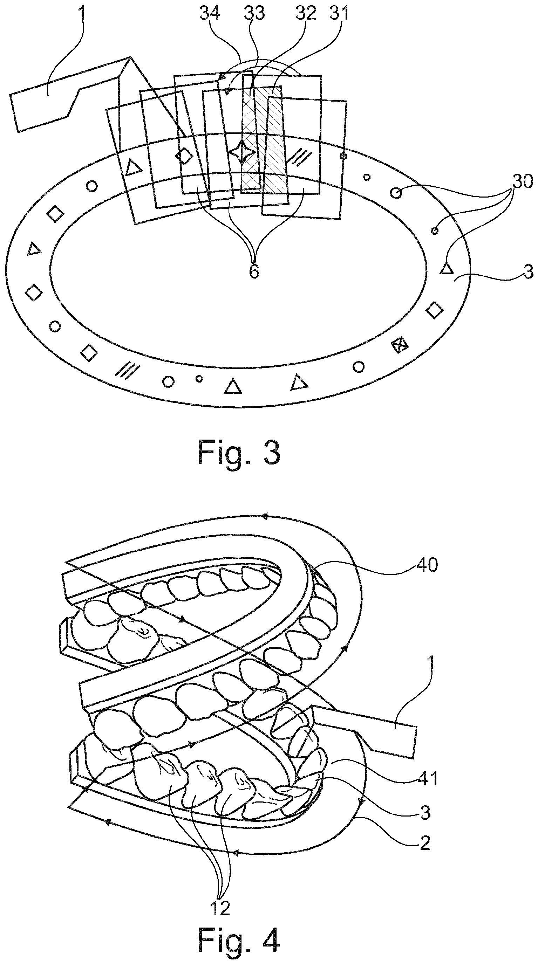

Die Fig. 3 zeigt ein im Vergleich zu Fig. 1 als solches nicht erfindungsgemäßes alternatives Referenzobjekt 3, das statt Zähnen dreidimensionale Modelle in Form von geometrischen Grundformen, wie Halbkugeln, Tetraedern oder Pyramiden, aufweist. Im Unterschied zu Fig. 1 sind die Aufnahmebereiche 6 so nahe beieinander angeordnet, dass jeder Aufnahmebereich sowohl mit dem vorangehenden Aufnahmebereich einen ersten Überlappungsbereich 31 aufweist, der mit einer breiten Schraffierung dargestellt ist, als auch einen zweiten Überlappungsbereich 32 mit dem Aufnahmebereich, der vor dem vorangehenden Aufnahmebereich angeordnet ist, wobei der zweite Überlagerungsbereich 32 mit einer schmaleren Schraffierung dargestellt ist. Dadurch wird jeder Aufnahmebereich zunächst mit dem vorangehenden Aufnahmebereich registriert, wie durch einen ersten Pfeil 33 angedeutet, und anschließend zusätzlich mit dem Aufnahmebereich registriert, der vor dem vorangehenden Aufnahmebereich angeordnet ist, was mit dem zweiten Pfeil 34 angedeutet ist. Auf diese Weise wird die Registrierung beim Zusammensetzen der einzelnen Aufnahmen 4 zu einer Gesamtaufnahme des Referenzobjekts 3 verbessert.

Die Fig. 4 zeigt eine Skizze zur Verdeutlichung einer Alternative des vorliegenden Verfahrens, bei dem im Vergleich zu Fig. 1 statt eines kreisförmigen Referenzobjekts ein herkömmlicher Artikulator mit einem Oberkiefermodell 40 und einem Unterkiefermodell 41 als Referenzmodell 3 verwendet wird. Die Vermessung mittels der Dentalkamera 1 erfolgt entlang einer Trajektorie 2, die um den gesamten Artikulator verläuft und wieder am Ausgangspunkt endet. Der Artikulator 3 wird wie in Fig. 1 Aufnahme für Aufnahme entlang der gesamten Trajektorie 2 vermessen. Anschließend werden die Aufnahmen registriert und eine Gesamtaufnahme erzeugt, wobei die doppelt vermessenen Objektbereiche des Referenzobjekts für den Vergleich um die Bestimmung des Messfehlers, wie oben zu Fig. 1 erläutert, herangezogen werden.

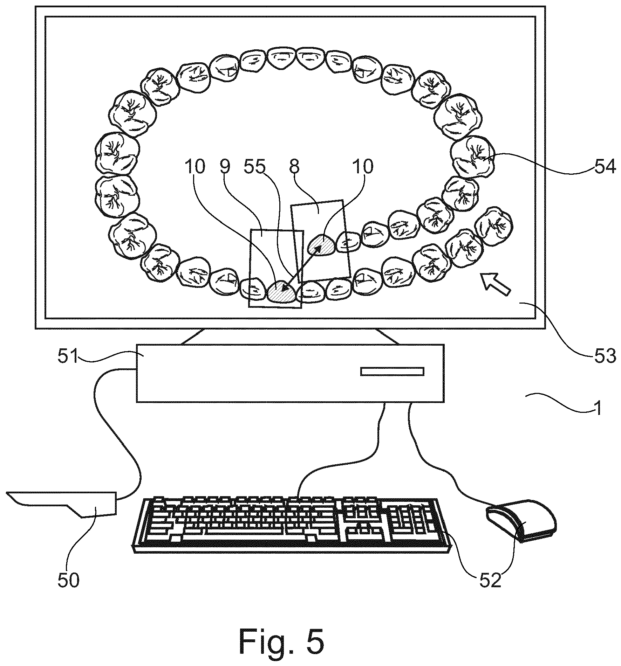

Die Fig. 5 zeigt zur Verdeutlichung des vorliegenden Verfahrens das gesamte Vermessungssystem 1 im Detail, umfassend ein Handstück 50, das die einzelnen Aufnahmen 4 auf Fig. 1 aufnimmt, während es relativ zum Referenzobjekt bewegt wird. Das Vermessungssystem umfasst weiterhin eine Bildanalyseeinheit 51, wie einen Computer, an den Bedienungselemente 52, wie die Tastatur und eine Maus angeschlossen sind, sowie eine Anzeigevorrichtung 53, wie einen Monitor. Die Bilddaten zu den Aufnahmen 4 werden vom Handstück 50 an die Bildanalyseeinheit 51 übermittelt und dort Aufnahme für Aufnahme registriert, sodass die Gesamtaufnahme 54 des Referenzobjekts 3 aus Fig. 1 erzeugt wird. Bei einer fehlerhaften Registrierung oder bei einer fehlerhaften Kalibrierung weicht die Form der Aufnahmen 4 aus Fig. 1 von der tatsächlichen Form des Referenzobjekts 3 ab. Die Abweichung setzt sich von Aufnahme zu Aufnahme der Registrierung fort, sodass eine erste Position eines doppelt vermessenen Objektbereichs 10 im ersten Aufnahmebereich 8 am Anfang der Vermessung und zu einer Position des Objektbereichs 10 im zweiten Aufnahmebereich 9 nach einem Umlauf einen Abstand 55 aufweist. Unter Verwendung des Abstands 55 kann dann der Messfehler des Vermessungssystems 1 bestimmt werden, der einen Registrierungsfehler und einen Kalibrierungsfehler umfasst. Falls der Abstand 55 klein genug ist und innerhalb festgelegter Toleranzgrenzen liegt, entspricht das geprüfte Vermessungssystem den Qualitätsanforderungen. Falls der Abstand 55 diese Toleranzgrenzen überschreitet, ist eine nachträgliche Kalibrierung oder Verbesserung des Registrierungsalgorithmus erforderlich.

### Bezugszeichen

- 1: Dentalkamera/Vermessungssystem
- 2: Trajektorie
- 3: Referenzobjekt
- 4: Aufnahmen
- 5: Aufnahmerichtung
- 6: Aufnahmebereiche
- 7: Überlappungsbereiche
- 8: erster Aufnahmebereich
- 9: letzer Aufnahmebereich
- 10: gemeinsamer Objektbereich
- 11: Pfeile
- 12: Zähne
- 20: erste Aufnahmerichtung
- 21: Zahnachse
- 22: zweite Aufnahmerichtung
- 31: erster Überlappungsbereich
- 32: zweiter Überlappungsbereich
- 33: erste Registrierung
- 34: zweite Registrierung
- 40: Oberkiefermodell
- 41: Unterkiefermodell
- 50: Handstück
- 51: Bildanalyseeinheit
- 52: Bedienungselement
- 53: Anzeigevorrichtung
- 54: Gesamtaufnahme
- 55: Abstand

## Patentansprüche

1. Verfahren zur Überprüfung eines Vermessungssystems (1), wobei mehrere dreidimensionale Aufnahmen (4) mittels des Vermessungssystems (1) von einem Referenzobjekt (3) aus unterschiedlichen Aufnahmerichtungen (5) aufgenommen werden, wobei das Referenzobjekt (3) eine geschlossene Form aufweist, wobei jede der dreidimensionalen Aufnahmen mindestens mit der vorangehenden Aufnahme registriert wird, wobei bei einer fehlerhaften Kalibrierung und/oder bei einer fehlerhaften Registrierung die einzelnen Aufnahmen (4) im Vergleich zur tatsächlichen Form des Referenzobjekts (3) verformt sind, sodass sich beim Zusammensetzen der einzelnen dreidimensionalen Aufnahmen (4) zu einer Gesamtaufnahme (54) die Verformung fortsetzt und dadurch die erzeugte Gesamtaufnahme (54) in ihren Abmessungen von den Abmessungen des Referenzobjekts (3) abweicht, wobei mindestens ein Objektbereich (10) des Referenzobjekts (3) am Anfang eines Umlaufs und am Ende des Umlaufs doppelt vermessen wird, wobei ein Abstand (55) zwischen einer ersten Position des Objektbereichs (10) in einer ersten Aufnahme (8) am Anfang des Umlaufs und einer zweiten Position des Objektbereichs (10) in einer zweiten Aufnahme (9) am Ende des Umlaufs in der Gesamtaufnahme (54) bestimmt wird, wobei unter Verwendung des Abstandes (55) ein Messfehler des Vermessungssystems bestimmt wird, der einen Kalibrierungsfehler und einen Registrierungsfehler umfasst, wobei vor der Durchführung der dreidimensionalen Aufnahmen (4) der Registrierungsfehler bestimmt wird, indem aus einem ersten ursprünglichen virtuellen 3D-Modell des Referenzobjekts (3) virtuelle einzelne Teilbereiche nacheinander ausgeschnitten werden, wobei jeder Teilbereich mit einem vorangehenden Teilbereich und mit einem nachfolgenden Teilbereich teilweise überlappend ist, wobei anschließend jeder Teilbereich mit mindestens einem vorangehenden Teilbereich registriert wird, sodass ein zweites virtuelles 3D-Modell des Referenzobjekts (3) nach der Registrierung erzeugt wird, wobei aus einer Abweichung zwischen dem ersten ursprünglichen 3D-Modell und dem zweiten 3D-Modell des Referenzobjekts (3) der Registrierungsfehler bestimmt wird, wobei der Kalibrierungsfehler bestimmt wird, indem der Registrierungsfehler vom gesamten Messfehler abgezogen wird.

2. Verfahren nach Anspruch 1, **dadurch gekennzeichnet, dass** das Referenzobjekt (3) ringförmig ist und Modelle von mehreren aneinandergereihten Zähnen (12) aufweist.

3. Verfahren nach Anspruch 2, **dadurch gekennzeichnet, dass** eine Trajektorie (2) des Vermessungssystems (1) beim Aufnehmen der Aufnahmen (4) aus unterschiedlichen Aufnahmerichtungen (5) kreisförmig um das ringförmige Referenzobjekt (3) verläuft.

4. Verfahren nach Anspruch 1, **dadurch gekennzeichnet, dass** das Referenzobjekt (3) einem herkömmlichen Artikulator mit einem Oberkiefer (40) und einem Unterkiefer (41) entspricht, der eine geschlossene Form aufweist.

5. Verfahren nach Anspruch 4, **dadurch gekennzeichnet, dass** eine Trajektorie (2) des Vermessungssystems (1) beim Aufnehmen der Aufnahmen (4) aus unterschiedlichen Aufnahmerichtungen (5) entlang der Form des Artikulators (3) verläuft.

6. Verfahren nach einem der Ansprüche 1 bis 5, **dadurch gekennzeichnet, dass** die Aufnahmerichtung einer letzten Aufnahme in der Reihenfolge der Aufnahmen (4) mit der Aufnahmerichtung einer ersten Aufnahme übereinstimmt.

7. Verfahren nach einem der Ansprüche 1 bis 5, **dadurch gekennzeichnet, dass** ein erster Aufnahmebereich (8) des Referenzobjekts (3) einer ersten Aufnahme in der Reihenfolge der Aufnahmen (4) zumindest teilweise mit einem Aufnahmebereich (9) einer der nachfolgenden Aufnahmen (4) übereinstimmt.

8. Verfahren nach Anspruch 1, **dadurch gekennzeichnet, dass** die Teilbereiche, die aus dem ersten ursprünglichen 3D-Modell ausgeschnitten werden, in ihren Abmessungen den Aufnahmebereichen (6) der einzelnen durchzuführenden Aufnahmen (4) entsprechen.

9. Verfahren nach einem der Ansprüche 1 bis 8, **dadurch gekennzeichnet, dass** jede Aufnahme mit der vorangehenden Aufnahme und mit der vorvorangehenden Aufnahme teilweise überlappend ist, wobei jede Aufnahme (4) mit der vorangehenden Aufnahme (4) und mit der vorvorangehenden Aufnahme (4) registriert wird.

10. Referenzmodell zur Überprüfung eines dentalen Vermessungssystems (1) zur Vermessung dreidimensionaler Aufnahmen (4), **dadurch gekennzeichnet, dass** das Referenzmodell aus mehreren aneinander gereihten Modellen von Zähnen (12) entlang des gesamten Umfangs des Referenzmodells aufgebaut ist und eine ringförmige Form aufweist.

## Claims

1. A method for checking a measuring system (1), wherein a plurality of three-dimensional recordings (4) of a reference object (3) are recorded from different recording directions (5) by means of the measuring system (1), wherein the reference object (3) has a closed shape, wherein each of the three-dimensional recordings is registered with at least the preceding recording, wherein in the case of a faulty calibration and/or in the case of a faulty registration, the individual recordings (4) are deformed compared with the actual shape of the reference object (3), so that the deformation continues when the individual three-dimensional recordings (4) are assembled to form an overall recording (54) and the generated overall recording (54) deviates in its dimensions from the dimensions of the reference object (3) as a result thereof, wherein at least one object region (10) of the reference object (3) is measured twice, at the beginning of a circuit and at the end of the circuit, wherein a distance (55) between a first position of the object region (10) in a first recording (8) at the beginning of the circuit and a second position of the object region (10) in a second recording (9) at the end of the circuit in the overall recording (54) is determined, wherein, using the distance (55), a measurement error of the measuring system is detected, said error comprising a calibration error and a registration error, wherein before performing the three-dimensional recordings (4) the registration error is determined by cutting out virtual individual sub-regions from a first original virtual 3D model of the reference object (3), wherein each sub-region partially overlaps a preceding sub-region and a subsequent sub-region, wherein subsequently each sub-region is registered with at least one preceding sub-region, so that a second virtual 3D model of the reference object (3) is generated after the registration, wherein the registration error is determined from a deviation between the first original 3D model and the second 3D model of the reference object (3), wherein the calibration error is determined by subtracting the registration error from the total measurement error.

2. The method according to claim 1, **characterised in that** the reference object (3) is annular and comprises models of a plurality of juxtaposed teeth (12).

3. The method according to claim 2, **characterised in that** a trajectory (2) of the measuring system (1) during recording of the recordings (4) from different recording directions (5) extends in a circle around the annular reference object (3).

4. The method according to claim 1, **characterised in that** the reference object (3) corresponds to a conventional articulator having an upper jaw (40) and a lower jaw (41) and having a closed shape.

5. The method according to claim 4, **characterised in that** a trajectory (2) of the measuring system (1) during recording of the recordings (4) from different recording directions (5) extends along the shape of the articulator (3).

6. The method according to any one of claims 1 to 5, **characterised in that** the recording direction of a final recording in the series of recordings (4) coincides with the recording direction of a first recording.

7. The method according to any one of claims 1 to 5, **characterised in that** a first recording region (8) of the reference object (3) of a first recording in the series of recordings (4) at least partially corresponds to a receiving region (9) of one of the subsequent recordings (4).

8. The method according to claim 1, **characterised in that** the sub-regions which are cut out of the first original 3D model correspond in their dimensions to the recording regions (6) of the individual recordings (4) to be performed.

9. The method according to any one of claims 1 to 8, **characterised in that** each recording partially overlaps the preceding recording and the previous preceding recording, wherein each recording (4) is registered with the preceding recording (4) and the previous preceding recording (4).

10. A reference model for checking a dental measuring system (1) for measuring three-dimensional images (4), **characterised in that** the reference model is constructed from a plurality of juxtaposed models of teeth (12) along the entire circumference of the reference model and has an annular shape.

## Revendications

1. Procédé de vérification d'un système de mesure (1), dans lequel une pluralité d'enregistrements tridimensionnels (4) sont pris, au moyen du système de mesure (1), d'un objet de référence (3) depuis différentes directions d'enregistrement (5), l'objet de référence (3) ayant une forme fermée, chacun des enregistrements tridimensionnels étant mis en registre au moins avec l'enregistrement précédent, les enregistrements individuels (4) étant déformés par rapport à la forme réelle de l'objet de référence (3) dans le cas d'un calibrage incorrect et/ou d'une mise en registre incorrecte, de sorte que lors de l'assemblage des enregistrements tridimensionnels individuels (4) en un enregistrement global (54), la déformation se poursuive et par conséquent l'enregistrement global généré (54) soit différent dans ses dimensions par rapport aux dimensions de l'objet de référence (3), au moins une zone (10) de l'objet de référence (3) étant mesurée deux fois au début d'un cycle et à la fin du cycle, une distance (55) entre une première position de la zone (10) dans un premier enregistrement (8) au début du cycle et une deuxième position de la zone (10) dans un second enregistrement (9) à la fin du cycle dans l'enregistrement global (54) étant déterminée ; à l'aide de la distance (55), une erreur de mesure du système de mesure étant déterminée, qui comprend une erreur de calibrage et une erreur de registre ; dans lequel, avant d'effectuer les enregistrements tridimensionnels (4), l'erreur de registre est déterminée ; en ce que des zones partielles individuelles virtuelles sont découpées les unes après les autres dans un premier modèle virtuel 3D d'origine de l'objet de référence (3) ; chaque zone partielle individuelle chevauchant par endroits une zone partielle précédente et une zone partielle suivante, chaque zone partielle étant ensuite mise en registre avec au moins une zone partielle précédente, de sorte qu'un deuxième modèle 3D virtuel de l'objet de référence (3) soit généré après la mise en registre, ; dans lequel, à partir d'un écart entre le premier modèle 3D d'origine et le deuxième modèle 3D de l'objet de référence (3), l'erreur de registre est déterminée, l'erreur de calibrage étant déterminée en soustrayant l'erreur de registre de l'erreur de mesure totale.

2. Procédé selon la revendication 1, **caractérisé en ce que** l'objet de référence (3) est annulaire et présente des modèles de plusieurs dents juxtaposées (12).

3. Procédé selon la revendication 2, **caractérisé en ce qu'**une trajectoire (2) du système de mesure (1) lors de l'enregistrement des enregistrements (4) à partir de différentes directions d'enregistrement (5) s'étend en cercle autour de l'objet de référence annulaire (3).

4. Procédé selon la revendication 1, **caractérisé en ce que** l'objet de référence (3) correspond à un articulateur classique ayant une mâchoire supérieure (40) et une mâchoire inférieure (41) ayant une forme fermée.

5. Procédé selon la revendication 4, **caractérisé en ce qu'**une trajectoire (2) du système de mesure (1) lors de l'enregistrement des enregistrements (4) à partir de différentes directions d'enregistrement (5) s'étend le long de la forme de l'articulateur (3).

6. Procédé selon l'une des revendications 1 à 5, **caractérisé en ce que** la direction d'enregistrement d'un dernier enregistrement dans l'ordre des enregistrements (4) coïncide avec la direction d'enregistrement d'un premier enregistrement.

7. Procédé selon l'une des revendications 1 à 5, **caractérisé en ce qu'**une première zone d'enregistrement (8) de l'objet de référence (3) d'un premier enregistrement dans l'ordre des enregistrements (4) coïncide au moins partiellement avec une zone d'enregistrement (9) d'un des enregistrements suivants (4).

8. Procédé selon la revendication 1, **caractérisé en ce que** les zones partielles qui ont été découpées du premier modèle 3D d'origine correspondent, dans leurs dimensions, aux zones d'enregistrement (6) des enregistrements individuels à effectuer (4).

9. Procédé selon l'une des revendications 1 à 8, **caractérisé en ce que** chaque enregistrement chevauche partiellement l'enregistrement précédent et l'enregistrement encore précédent, chaque enregistrement (4) étant mis en registre avec l'enregistrement précédent (4) et l'enregistrement encore précédent (4).

10. Modèle de référence pour vérifier un système de mesure dentaire (1) pour mesurer des enregistrements tridimensionnels (4), **caractérisé en ce que** le modèle de référence est construit à partir d'une pluralité de modèles de dents (12) alignés les uns à côté des autres sur toute la circonférence du modèle de référence et présente une forme annulaire.
